# EUROPÄISCHE PATENTSCHRIFT

(11) **EP 0 756 475 B1**
(45) Veröffentlichungstag und Bekanntmachung des Hinweises auf die Patenterteilung: **30.12.1998**
(21) Anmeldenummer: 95916640.6
(22) Anmeldetag: 12.04.1995
(51) Int. Cl.: A61F 2/02, A61F 2/12, A61L 27/00

(54) **MEDIZINISCHE IMPLANTATE AUS FORMKÖRPERN**
MEDICAL IMPLANTS MADE OF MOULDINGS
IMPLANT MEDICAL REALISE A PARTIR DE CORPS MOULES

(30) Priorität: 22.04.1994 DE 4414103
(43) Veröffentlichungstag der Anmeldung: 05.02.1997
(73) Patentinhaber: Reinmüller, Johannes, Dr. med., W-65189 Wiesbaden (DE)
(72) Erfinder: Reinmüller, Johannes, Dr. med., W-65189 Wiesbaden (DE)
(74) Vertreter: Huber, Bernhard, Dipl.-Chem.
(86) Internationale Anmeldenummer: EP9501357
(87) Internationale Veröffentlichungsnummer: WO9528894

(56) Entgegenhaltungen:
- EP-A- 0 051 954
- EP-A- 0 057 033
- EP-A- 0 322 194
- US-A- 4 200 110
- US-A- 4 657 820
- US-A- 5 358 521

## Beschreibung

Diese Erfindung betrifft Kunststoffimplantate für medizinische Zwecke und insbesondere Implantate, umfassend dünne faltbare Formkörper.

In der Human- und Tiermedizin werden angeborene oder erworbene Organ- oder Weichteildefekte mit Kunststoffimplantaten (KI) ausgefüllt, die operativ in den Organismus eingebracht werden. Im Gegensatz zu orthopädischen Implantaten oder zu künstlichen Organen, die ebenfalls in den Organismus implantiert werden, ist es die Aufgabe der vorgenannten Kunststoffimplantate, einen Volumenersatz von Weichgeweben zur Verfügung zu stellen und in einer besonderen Ausführungsform die Dehnung und Erweiterung von Gewebe, insbesondere von Hautgewebe, zu bewirken.

Typische Beispiele von KI umfassen den Ersatz von fehlendem Brustdrüsengewebe durch Silikonkissen, die vor oder hinter dem Brustmuskel implantiert werden oder Implantate zum Ersatz fehlender Hoden. Beispiele für Implantate zur Dehnung und Erweiterung von Gewebe zum Aufbau von Weichteilen im Gesicht, an den Extremitäten oder am Körperstamm sind herkömmliche Expander, wie z.B. der Becker-Expander, wie in Joseph G. McCarthy, Plastic Surgery (1990), W.B. Sounders Comp., Band 1, Kapitel 13, S. 486 beschrieben.

Um eine möglichst natürlich wirkende Rekonstruktion von Weichteilgeweben zu erreichen, werden für Kunststoffimplantate bevorzugt Stoffe verwendet, deren mechanische Eigenschaften den natürlichen Geweben sehr nahe kommen. Weiterhin ist es erforderlich, daß das verwendete Implantatmaterial physiologisch verträglich ist, keine toxischen Wirkungen hervorruft und eine extreme chemische und mechanische Langzeitstabilität aufweist. Elastisch verformbare Kunststoffe erfüllen diese Anforderungen im allgemeinen, insbesondere in Form von Gelen. Aufgrund der hervorragenden chemischen Stabilität hat sich die Verwendung von Dimethylsiloxanpolymeren (PDMS) als besonders günstig erwiesen.

Herkömmliche Implantate umfassen eine äußere Hülle aus Silikonmaterial in deren Inneres ein hochpolymeres Gel, z.B. ein PDMS-Gel eingeführt wird, oder wäßrige Lösungen. Die Zugabe des Gels bzw. der wäßrigen Lösung kann vor der Implantation erfolgen oder im Falle der wäßrigen Lösung nachträglich durch ein von der Körperoberfläche zugängliches Ventil. Derartige Implantate sind beispielsweise in US-Pat Nr. 4428364, 4574780, 4671255 und 4840615 offenbart.

Herkömmliche Produkte, die Gele und insbesondere Produkte, die PDMS-Gele verwenden, weisen jedoch den Nachteil auf, daß im Fall einer Verletzung der äußeren Hülle, z.B. bei einem Unfall, eine Infiltration des umliegenden Gewebes mit (PDMS-)Gel unter hohem Druck erfolgt. Eine solche Situation kann nachfolgend durch operative Maßnahmen kaum beherrscht werden. Weiterhin wird durch neuere Untersuchungen die pysiologische Verträglichkeit der PDMS-Implantate in Frage gestellt. Es wird angenommen, daß niedermolekulare Bestandteile des PDMS-Gels durch die äußere Hülle diffundieren und damit in das umliegende Gewebe gelangen, wobei langfristig unter Umständen irreversible Gesundheitsschäden hervorgerufen werden. Durch die Einrichtung von Sperrschichten kann diese Penetration zwar reduziert, aber nicht vollständig unterbunden werden.

Versuche, die PDMS-Gele aufgrund oben genannter Nachteile durch andere Materialien zu ersetzen, führten bisher zu keinem befriedigenden Ergebnis:

Physiologische Salzlösungen sind nicht geeignet, da sie als Füllmaterial eines Kunststoffimplantats unerwünschte mechanische und druckelastische Eigenschaften vermitteln. Außerdem verlieren solche Implantate im Laufe von wenigen Jahren ihre Flüssigkeitsvolumen, da die äußere Hülle aus PDMS-Kautschuk keine Barriere für niedermolekulare Stoffe darstellt.

Die Verwendung anderer Ersatzstoffe, wie z.B. Hydrogele, Dextrane, Hylane, Gelatine oder Polyvinylpyrolidon (PVP) ist aufgrund ihrer mangelhaften Langzeitstabilität nachteilig oder aufgrund ihrer Toxizität limitiert.

EP-0 322 194 lehrt ein Implantat, bestehend aus einer äußeren Hülle, die der Bildung von Narbengewebe entgegenwirken und das Einwachsen von normalem Gewebe unterstützen soll, und einer Füllung, die dem Implantat komprimierbare und somit gewebeähnliche Eigenschaften verleiht. Die Füllung wird geeignet durch eine entsprechende Innenstruktur gebildet oder alternativ können auch Formkörper eingsetzt werden die kugelförmig dargestellt sind und bevorzugt selbst eine Innenstruktur aufweisen, die ihnen Komprimierbarkeit verleiht.

Aufgabe der vorliegenden Erfindung war es nunmehr, ein Kunststoffimplantat zur Verfügung zu stellen, das die Vorteile der PDMS-Gele gewährleistet, nämlich druckelastische und somit gewebeähnliche Eigenschaften sowie chemische Langzeitstabilität, aber gleichzeitig die oben erwähnten Nachteile nicht aufweist, nämlich Diffusion niedermolekularer toxischer Bestandteile in das umliegende Gewebe sowie Infiltration des umliegenden Gewebes mit PDMS-Gel bei Verletzung der äußeren Hülle.

Diese Aufgabe wird gemäß vorliegender Erfindung gelöst durch ein Implantat für medizinische Zwecke auf Basis eines physiologisch verträglichen Kunststoffs, das aus dünnen faltbaren Formkörpern mit einer für ein fluides Gleitmittel benetzbaren Oberfläche besteht.

Das Volumen des Implantats wird dabei normalerweise durch die dünnen Formkörper bestimmt, während das Gleitmittelvolumen untergeordnet ist. Zum Aufbau eines erfindungsgemäßen Implantats werden als Formkörper beispielsweise dünne Folien verwendet, wobei eine Vielzahl aufeinanderliegender Schichten ein Implantat der gewünschten Dicke ergibt.

Die gewünschten gewebeähnlichen elastischen Eigenschaften des Implantats der vorliegenden Erfindung werden im wesentlichen durch die Verschiebbarkeit aneinandergrenzender Schichten der dünnen faltbaren Formkörper erzielt, wobei die Gleitwirkung durch ein geeignetes Gleitmittel vermittelt bzw. begünstigt wird.

Die Formkörper der vorliegenden Erfindung können entweder direkt ohne eine äußere Hüllschicht in eine präformierte Implantatloge im Gewebe eingebracht werden, oder nach Art herkömmlicher Implantate von einer äußeren Hülle umgeben sein, wobei die Gele herkömmlicher Implantate durch die Formkörper der vorliegenden Erfindung ersetzt werden.

Als Material für die Formkörper kommen alle physiologisch verträglichen Kunststoffe in Betracht. Unter den Begriff "physiologisch verträgliche Kunststoffe" werden in dieser Anmeldung allgemein natürlich vorkommende und künstlich hergestellte Materialien bzw. Biomaterialien verstanden, die physiologisch verträglich sind. In einer besonderen Ausführungsform bestehen die Formkörper aus Silikonkautschuk, wobei Silikonkautschuk aus Polydimethylsiloxan bevorzugt ist. In einer anderen Ausführungsform werden Formkörper aus Polysacchariden verwendet, wobei Formkörper aus Cuprophanfolie eine bevorzugte Ausführungsform darstellen. Die Erfindung beschränkt sich jedoch nicht auf die vorgenannten Materialien, und andere physiologisch verträgliche Kunststoffe und Biomaterialien, die zu dünnen Formkörpern verarbeitet werden können, sind Bestandteil der vorliegenden Erfindung.

Die dünnen faltbaren Formkörper der Erfindung können in verschiedenen Ausführungsformen bereitgestellt werden. Bevorzugt ist die Verwendung einer Folie, die auf geeignete Weise gefaltet wird bzw. von mehreren aufeinanderliegenden Folienlagen, die geeignet zugeschnitten werden, um die Form des endgültigen Implantats auszumachen. Es wurde festgestellt, daß die Verwendung von Folien mit einer Dicke von etwa 5 bis etwa 500 µm günstig ist, wobei 10 bis 200 µm bevorzugt und 30 bis 70 µm besonders bevorzugt sind. Bei einer anderen Ausführungsform der Erfindung werden die Formkörper als kapillare Röhren oder in schlauchartiger Ausgestaltung zur Verfügung gestellt. In einzelnen Fällen kann es besonders günstig sein, eine Kombination von zwei oder mehreren Ausführungsformen zu verwenden. Solche Kombinationen werden von der vorliegenden Erfindung ebenfalls umfaßt.

Die Implantate der Erfindung können nach Einbringen in das Gewebe postoperativ kontrolliert werden. Beispielsweise sind PDMS-Folien für weiche Röntgenstrahlung dichter als Gewebe, womit die Lage eines Implantats postoperativ durch Röntgenuntersuchung kontrolliert werden kann. Erfindungsgemäße Implantate, die Formkörper aus PDMS umfassen, sind somit ohne weitere Zusatzstoffe der postoperativen Untersuchung zugänglich. In anderen Fällen kann durch den Zusatz geeigneter von außen nachweisbarer Stoffe, wie beispielsweise Röntgenkontrastmittel im Kunststoff, eine spätere Überprüfung der Implantate auf einfache Weise ermöglicht werden.

Die gewebsähnlichen Eigenschaften der Implantate der vorliegenden Erfindung beruhen zu einem wesentlichen Teil auf der Gleitfähigkeit von aneinandergrenzenden Schichten der dünnen Formkörper, wobei die Gleitfähigkeit durch ein geeignetes fluides Gleitmittel begünstigt wird.

Mit dem Begriff "fluides Gleitmittel" werden in dieser Anmeldung allgemein flüssige und fluide Phasen bezeichnet, die physiologisch verträglich sind und die Verschiebbarkeit der Oberflächen aneinandergrenzender Formkörper ermöglichen und/oder verbessern. Die Art des verwendeten Gleitmittels ist lediglich von der Oberfläche des verwendeten Formkörpers abhängig. Im Fall einer hydrophilen Oberfläche der Formkörper wird als Gleitmittel ebenfalls ein hydrophiles Fluid verwendet. Durch hydrophile Wechselwirkungen vermitteltes Gleiten ist die bevorzugte Ausführungsform, wobei in diesem Fall die Verwendung einer wäßrigen Flüssigkeit als Gleitmittel besonders bevorzugt ist. Entsprechend kann die Gleitfähigkeit auch durch Verwendung hydrophober Oberflächen/Gleitmittel hervorgerufen werden, wobei als hydrophobe Gleitmittel Fette oder Öle bevorzugt sind. Hydrophil/hydrophob vermittelte Gleitvorgänge, z.B. durch Liposome oder Micellen ampholytischer Stoffe, sind ebenfalls Gegenstand dieser Erfindung.

In vielen Fällen kann es wünschenswert sein, die Oberflächen der dünnen Formkörper durch eine Hydrophobierung oder Hydrophilisierung den Eigenschaften des verwendeten Gleitmittels anzupassen, um ihre Gleitfähigkeit weiter zu verbessern. Entsprechende Verfahren sind dem Fachmann wohlbekannt. In DE-OS 4216271 wird beispielsweise die Hydrophilisierung von Silikonoberflächen durch Ätzen mit einer Lösung aus KOH in Methanol beschrieben. EP-A-0 086 186 beschreibt die Hydrophilisierung von aminhaltigen Oberflächen mit Substanzen, die 2-Amino-2-deoxyglycopyranosil-Reste enthalten. Geeignete Verfahren zur Hydrophobierung umfassen beispielsweise das Aufbringen von Silikonschichten oder von Wachsen.

Die Verwendung von Formkörpern mit hydrophilisierter Oberfläche stellt eine besonders günstige Anwendung der Formkörper der vorliegenden Erfindung dar. Durch die Hydrophilisierung wird das Gleitmittel, vorzugsweise Wasser, im Inneren der Implantate gebunden und somit ein Volumenverlust durch Diffusion wie bei herkömmlichen Implantaten auf Flüssigkeitsbasis wirksam verhindert. Die Implantate der vorliegenden Erfindung können in dieser Ausführungsform gegebenenfalls ohne Zusatz von Gleitmittel implantiert werden. In diesem Fall wird das Gleitmittel aus dem umgebenden Gewebe zur Verfügung gestellt, da Gewebswasser ins Innere der Implantate und, falls vorhanden, durch die äußere Hülle diffundieren kann.

In einer anderen besonderen Ausführungsform enthalten die Implantate der vorliegenden Erfindung quellfähige Materialien. Geeignete quellfähige Materialien zur Herstellung der erfindungsgemässen Formkörper sind im allgemeinen alle pyhsiologisch verträglichen Stoffe, die bei Kontakt mit einer geeigneten Flüssigkeit quellfähig sind. Besonders vorteilhaft ist die Verwendung von Polysacchariden, wobei das Polysaccharid Cuprophanfolie besonders bevorzugt ist. In diesem Fall ist Wasser sowohl Quell- als auch Gleitmittel.

Die Verwendung quellfähiger Materialien ist jedoch nicht auf die Formkörper beschränkt, sondern anstelle oder zusätzlich zu quellfähigen Formkörpern kann ein quellfähiges Gleitmittel verwendet werden. Geeignete quellfähige Gleitmittel sind beispielsweise wasserfreie Polysaccharide, Glycosaminoglycane, Dextrane und dgl.. Hyaluronsäure ist bevorzugt. Weitere geeignete quellfähige Materialien sind dem Fachmann bekannt.

Weiterhin enthalten die Implantate der vorliegenden Erfindung je nach Bedarf bzw. Verwendungszweck andere Zusätze, wie z.B. Farbstoffe, Antioxidantien, Stabilisatoren, Desinfizienzien, Antibiotika, Salze und dgl..

Die Implantate der vorliegenden Erfindung werden entweder als Weichteilersatz oder zur Expansion von umliegendem Gewebe verwendet. Die Kunststoffimplantate werden gewöhnlich von einer äußeren elastischen Hülle umgeben sein, wobei die Form der Implantate von den Ausmaßen der äußeren Hülle bestimmt wird. Durch Über- oder Unterfüllung der äußeren Hüllen mit den erfindungsgemäßen Formkörpern werden mehr oder weniger prallelastische Eigenschaften erzeugt. Das Gleitmittel füllt üblicherweise nur die kapillaren Spalten, die zwischen den Lagen des Füllmaterials vorkommen und trägt somit nur zu einem geringen Anteil zum Füllvolumen bei. Alternativ dazu kann ein erfindungsgemäßes Implantat mit quellfähigen Formkörpern und/oder Gleitmittel unterfüllt werden, wobei das Endvolumen des Implantats im Voraus durch die Füllmenge und die gegebene Quellfähigkeit der Materialien bestimmt ist, aber erst nach Implantation erreicht wird.

Eine besondere Verwendungsform der Implantate der vorliegenden Erfindung stellen sogenannte Gewebeexpander dar, die erst nach Implantation bzw. Einheilung des Expanders nachträglich auf ihr endgültiges Volumen gebracht werden. Dabei wird eine Volumenänderung durch Zugabe oder Entnahme des Gleitmittels reguliert. Ähnlich wie bei dem bekannten Becker-Expander kann bei Bedarf der Ventilmechanismus nach Erreichen des gewünschten Expandervolumens entfernt werden, wodurch der Expander mit der erfindungsgemäßen Füllung zum permanenten Implantat wird.

Die Vorteile der vorliegenden Erfindung bestehen im wesentlichen darin, daß die dünnen faltbaren Formkörper, die das erfindungsgemäße Füllmaterial der Implantate darstellen, die gleiche Langzeitstabilität wie das herkömmlich äußere Hüllmaterial aufweisen. Dadurch wird die Abgabe toxischer Substanzen über einen langen Zeitraum wesentlich reduziert. Weiterhin wird bei einer Verletzung der äußeren Hülle das Füllmateriel nicht wie im Fall der herkömmlichen PDMS-Gele in unkontrollierter Weise im umgebenden Gewebe dispergiert, sondern kann operativ einfach und vollständig entfernt werden. Freigesetztes Gleitmittel wird ohne schädliche Folgen für den Organismus vom umgebenden Gewebe aufgenommen bzw. vermischt sich mit dem Gewebswasser. Bei einer bevorzugten Ausführungsform, der Verwendung von Formkörpern aus hydrophilisierter PDMS-Folie und Wasser als Gleitmittel, ist weiterhin die einfache Sterilisierbarkeit im Autoklaven ein großer Vorteil.

Die folgenden Beispiele veranschaulichen die vorliegende Erfindung.

### BEISPIEL 1

### Hydrophilisierung von Siliconoberflächen

Siliconkautschukfolien der Stärken 0,02 mm, 0,2 mm und 0,3 mm werden durch Ätzen mit einer Lösung aus 5,7 g KOH ad 100 ml Methanol, wie in DE-OS 4216271 beschrieben, hydrophilisiert.

### BEISPIEL 2

### Mammaimplantat

Eine Hülle aus Silikonkautschuk mit eliptoidem Querschnitt und rundem Längsschnitt mit einer Wandstärke von 0,2 mm wird hergestellt. Durch eine Öffnung wird hydrophilisierte Silikonkautschukfolie der Stärke 0,02 mm aus Beispiel 1 eingebracht. Die äußere Hülle besitzt in entspanntem Zustand ein Fassungsvermögen von 200 ml. Die Menge des zugegebenen Füllmaterials entspricht einem Volumen von 190 ml. Weiterhin wird ins Innere ein Volumen von 10 ml Wasser zugegeben. Die Füllöffnung wird dicht verschlossen. Das so hergestellte Mammaimplantat wird in einer Vakuumkammer entgast und danach im Autoklaven sterilisiert.

### BEISPIEL 3

### Hautexpander

Eine Silikonkautschukhülle in Quaderform mit abgerundeten Ecken und Kanten wird hergestellt. Sie wird innen und außen wie in Beispiel 1 hydrophilisiert. Durch eine Öffnung wird Folienmaterial aus Celluloseacetat der Stärke 0,01 mm zugegeben. Zwischen den einzelnen Lagen der Folie wird ein Puder aus wasserfreier quervernetzter Hyaluronsäure eingestreut. Die Hülle wird dicht verschlossen, und das Implantat wird autoklaviert. Nach Implantation des so erzeugten Implantats in den Organismus, z.B. im subcutanen Gewebe, dringt durch die Silikonkautschukhülle Wasser in das Innere und führt zur Quellung des Füllmaterials. Das Implantat dehnt sich räumlich aus und expandiert dabei die Haut. Die Größenzunahme im zeitlichen Verlauf wird durch den Wassereinstrom gesteuert. Das Endvolumen des Hautexpanders wird aufgrund der Quellfähigkeit des Füllmaterials durch die eingebrachte Füllmenge voraus definiert.

## Patentansprüche

1. Implantat für die Rekonstruktion von Weichteilgewebe auf Basis eines physiologisch verträglichen Kunststoffs,
**dadurch gekennzeichnet,**
daß es aus dünnen faltbaren Formkörpern aus Folie mit einer Dicke von 10 bis 200 µm mit einer für ein fluides Gleitmittel benetzbaren Oberfläche besteht.

2. Implantat nach Anspruch 1,
**dadurch gekennzeichnet,**
daß es von einer Hülle umgeben ist.

3. Implantat nach Anspruch 1 oder 2,
**dadurch gekennzeichnet,**
daß es aus schlauchförmigen Folien besteht.

4. Implantat nach einem der Ansprüche 1 bis 3,
**dadurch gekennzeichnet,**
daß es eine hydrophilisierte Oberfläche aufweist.

5. Implantat nach Anspruch 4,
**dadurch gekennzeichnet,**
daß es als Gleitmittel ein wäßriges Fluid enthält.

6. Implantat nach einem der vorhergehenden Ansprüche,
**dadurch gekennzeichnet,**
daß der dünne faltbare Formkörper bei Kontakt mit einer geeigneten Flüssigkeit quellfähig ist.

7. Implantat nach einem der vorhergehenden Ansprüche,
**dadurch gekennzeichnet,**
daß das Gleitmittel quellfähig ist.

8. Implantat nach Anspruch 7,
**dadurch gekennzeichnet**,
daß das Gleitmittel ein Polysaccharid oder Glucosaminoglycan ist.

9. Implantat nach einem der Ansprüche 1 bis 3,
**dadurch gekennzeichnet,**
daß der dünne faltbare Formkörper eine hydrophobierte Oberfläche aufweist.

10. Implantat nach Anspruch 9,
**dadurch gekennzeichnet,**
daß es als fluides Gleitmittel Fett oder Öl enthält.

11. Implantat nach einem der vorhergehenden Ansprüche,
**dadurch gekennzeichnet,**
daß der Kunststoff ein Silikonkautschuk ist.

12. Implantat nach Anspruch 11,
**dadurch gekennzeichnet,**
daß der Silikonkautschuk aus Polydimethylsiloxan besteht.

13. Implantat nach einem der Ansprüche 1 bis 8,
**dadurch gekennzeichnet,**
daß der Kunststoff ein Polysaccharid ist.

14. Implantat nach Anspruch 6, 7 und 13,
**dadurch gekennzeichnet,**
daß das Polysaccharid Cuprophanfolie ist.

15. Implantat nach einem der vorhergehenden Ansprüche,
**dadurch gekennzeichnet,**
daß der Kunststoff des Formkörpers ein Röntgenkontrastmittel oder einen Farbstoff inkorporiert enthält.

## Claims

1. Implant for the reconstruction of soft tissue based on a physiologically compatible plastic,
**wherein**
it is composed of thin foldable structural elements made of foil having a thickness of 10 to 200 µm and a surface that is wettable by a fluid lubricant.

2. Implant as claimed in claim 1,
**wherein**
it is surrounded by a covering.

3. Implant as claimed in claim 1 or 2,
**wherein**
it is composed of tubular foils.

4. Implant as claimed in one of the claims 1 to 3,
**wherein**
it has a hydrophilised surface.

5. Implant as claimed in claim 4,
**wherein**
it contains an aqueous fluid as the lubricant.

6. Implant as claimed in one of the previous claims,
**wherein**
the thin foldable structural element is capable of swelling when contacted with a suitable liquid.

7. Implant as claimed in one of the previous claims,
**wherein**
the lubricant is swellable.

8. Implant as claimed in claim 7,
**wherein**
the lubricant is a polysaccharide or glucosaminoglycan.

9. Implant as claimed in one of the claims 1 to 3,
**wherein**
the thin foldable structural element has a hydrophobised surface.

10. Implant as claimed in claim 9,
**wherein**
it contains fat or oil as the fluid lubricant.

11. Implant as claimed in one of the previous claims,
**wherein**
the plastic is a silicone rubber.

12. Implant as claimed in claim 11,
**wherein**
the silicone rubber is composed of polydimethylsiloxane.

13. Implant as claimed in one of the claims 1 to 8,
wherein
the plastic is a polysaccharide.

14. Implant as claimed in claim 6, 7 and 13,
**wherein**
the polysaccharide is cuprophane foil.

15. Implant as claimed in one of the previous claims,
**wherein**
an X-ray contrast medium or a dye is incorporated into the plastic of the structural element.

## Revendications

1. Implant pour la reconstruction de tissu de partie molle à base d'une matière synthétique physiologiquement acceptable, caractérisé en ce qu'il consiste en corps façonnés pliables minces en feuille d'une épaisseur de 10 à 200 µm avec une surface mouillable pour un lubrifiant fluide.

2. Implant selon la revendication 1 caractérisé en ce qu'il est entouré par une enveloppe.

3. Implant selon la revendication 1 ou 2 caractérisé en ce qu'il consiste en feuilles en forme de tuyaux.

4. Implant selon l'une des revendications 1 à 3 caractérisé en ce qu'il comporte une surface rendue hydrophile.

5. Implant selon la revendication 4 caractérisé en ce qu'il contient un fluide aqueux comme lubrifiant.

6. Implant selon l'une des revendications précédentes caractérisé en ce que le corps façonné pliable mince est capable de gonfler par contact avec un liquide approprié.

7. Implant selon l'une des revendications précédentes caractérisé en ce que le lubrifiant est capable de gonfler.

8. Implant selon la revendication 7 caractérisé en ce que le lubrifiant est un polysaccharide ou un glucosaminoglycane.

9. Implant selon l'une des revendications 1 à 3 caractérisé en ce que le corps façonné pliable mince présente une surface rendue hydrophobe.

10. Implant selon la revendication 9 caractérisé en ce qu'il contient comme lubrifiant fluide une graisse ou une huile.

11. Implant selon l'une des revendications précédentes caractérisé en ce que la matière synthétique est un caoutchouc de silicone.

12. Implant selon la revendication 11 caractérisé en ce que le caoutchouc de silicone consiste en polydiméthylsiloxane.

13. Implant selon l'une des revendications 1 à 8 caractérisé en ce que la matière synthétique est un polysaccharide.

14. Implant selon les revendications 6, 7 et 13 caractérisé en ce que le polysaccharide est une feuille de cuprophane.

15. Implant selon l'une des revendications précédentes caractérisé en ce que la matière synthétique du corps façonné contient à l'état incorporé un agent de contraste pour rayons X ou un colorant.
